# FASCICULE DE BREVET EUROPEEN

(11) **EP 0 725 600 B1**
(45) Date de publication et mention de la délivrance du brevet: **03.06.1998**
(21) Numéro de dépôt: 94931629.3
(22) Date de dépôt: 25.10.1994
(51) Int. Cl.: A61B 17/34, A61M 1/08

(54) **INSTRUMENT CHIRURGICAL POUR LE TRAITEMENT D'UN KYSTE HYDATIQUE**
CHIRURGISCHES INSTRUMENT ZUR BEHANDLUNG EINER WASSER ENTHALTENDEN ZYSTE
SURGICAL INSTRUMENT FOR TREATING A HYDATID CYST

(30) Priorité: 26.10.1993 FR 9312744
(43) Date de publication de la demande: 14.08.1996
(73) Titulaire: ETHNOR, F-92523 Neuilly sur Seine Cédex (FR)
(72) Inventeur: FOURATI, Maher, Tunis (TN)
(74) Mandataire: Texier, Christian
(86) Numéro de dépôt international: FR9401240
(87) Numéro de publication internationale: WO9511634

(56) Documents cités:
- EP-A- 0 450 886
- WO-A-85/00009
- DE-A- 3 321 621
- FR-A- 996 531
- FR-A- 1 057 683
- GB-A- 1 048 558
- US-A- 2 012 363
- DATABASE WPI Derwent Publications Ltd., London, GB; AN 84-119809 & SU,A,1 034 746 (UKR PHYSICS INST)

## Description

La présente invention concerne le domaine des instruments chirurgicaux.

Plus précisément la présente invention concerne un instrument chirurgical pour le traitement de collections liquidiennes dans le corps humain.

La présente invention trouve tout particulièrement application dans la stérilisation et l'aspiration de kystes hydatiques.

D'une façon plus générale cependant la présente invention peut s'appliquer au traitement de toute "collection liquidienne", notamment les kystes liquidiens, tels que les kystes hydatiques ou kystes suspects, par exemple les kystes de l'ovaire, et les collections purulentes ou abcès.

La technique classique pour traiter chirurgicalement les kystes hydatiques consiste :
- à réaliser une voie d'abord dans le corps humain, de dimension suffisante pour accèder au kyste et à tout l'environnement de celui-ci,
- à envelopper le kyste ainsi rendu accessible à l'aide de compresses imbibées d'un liquide scolicide,
- à ponctionner le kyste,
- puis à stériliser le contenu du kyste en vidant celui-ci et en le remplissant, le plus souvent simultanément, d'un liquide stérilisant, à l'aide d'aiguilles de ponction, et
- lorsque la poche formant le kyste est ainsi stérilisée de façon satisfaisante, à éliminer par aspiration la membrane qui constitue la paroi du kyste.

Cette technique classique ne donne pas totalement satisfaction.

En premier lieu, les dimensions de la voie d'abord requises pour opérer le traitement chirurgical dans de bonnes conditions imposent une période d'hospitalisation importante, entrainent parfois des complications opératoires et post-opératoires, génèrent des cicatrices, conduisent à une intervention peu confortable pour les patients, accompagnée parfois de douleurs notables exigeant une consommation importante de médicaments d'appoint, notamment de médicaments antalgiques-antidouleurs.

En second lieu, il faut noter que la technique classique exige des précautions rigoureuses du fait que la moindre fuite, dans le corps humain, de liquide infecté provenant du kyste hydatique peut induire la croissance ultérieure de nouveaux kystes nécessitant de nouvelles interventions.

Le document EP-A-450886 enseigne un instrument endoscopique pour l'aspiration de fluide dans un kyste ovarien. Cet instrument comprend une canule pourvue à son extrémité distale d'un embout d'aspiration, et conçue pour être raccordée à une source à dépression à son extrémité proximale, et une aiguille creuse de ponction. La ponction du kyste est réalisée en déplaçant la pointe de l'aiguille dans le kyste au-delà de l'embout d'aspiration. La mise en dépression de la canule a pour but d'éviter toute fuite de liquide.

La présente invention a maintenant pour but de perfectionner les instruments chirurgicaux de traitement de collections liquidiennes, en particulier de kystes hydatiques.

La présente invention a en particulier pour but de proposer un nouvel instrument chirurgical qui permette un traitement complet sous endoscopie.

Ces buts sont atteints dans le cadre de la présente invention grâce à un instrument chirurgical pour le traitement d'une collection liquidienne, en particulier pour la stérilisation et l'ablation d'un kyste hydatique, du type comprenant, conforément au document EP-A-450886 :
- une canule pourvue d'une ventouse à son extrémité distale et conçue pour être raccordée à une source à dépression à son extrémité proximale,
- et au moins une aiguille creuse de ponction, dont l'extrémité distale est entourée par la canule, apte à être déplacée à translation par rapport à la canule entre une première position dans laquelle la pointe de l'aiguille est en retrait dans la ventouse ou la canule et une seconde position, de ponction, dans laquelle la pointe de l'aiguille fait saillie au delà de la ventouse, caractérisé par le fait qu'il comprend deux aiguilles creuses et au moins un canal opérateur séparé des aiguilles, dans la canule, et adapté pour recevoir au moins un outil d'intervention.

D'autre caractéristiques, buts et avantages de la présente invention apparaitront à la lecture de la description détaillée qui va suivre et en regard des dessins annexés donnés à titre d'exemples non limitatifs et sur lesquels :
- la figure 1 représente une vue latérale extérieure d'un instrument conforme à la présente invention avant assemblage de la ventouse sur la canule,
- la figure 2 représente une vue en coupe axiale de la canule conforme à la présente invention,
- la figure 3 représente une vue en coupe axiale similaire de la canule équipée de deux aiguilles de ponction,
- les figures 4 à 7 représentent schématiquement quatre étapes successives d'utilisation de l'instrument chirurgical conforme à la présente invention représenté sur les figures 1 à 3,
- la figure 8 représente une vue en coupe axiale longitudinale d'un corps tubulaire externe de la canule susceptible d'être utilisé dans le cadre d'une variante de réalisation de la présente invention,
- la figure 9 représente une vue en coupe similaire du même corps tubulaire externe de la canule placé sur l'instrument conforme aux figures 1 à 3,
- la figure 10 représente une variante du corps tubulaire externe équipé d'une ventouse,
- la figure 11 représente une vue en coupe d'une ventouse conforme à la présente invention,
- la figure 12 représente une vue en plan d'une plaque de matériau absorbant susceptible d'être utilisée en combinaison avec l'instrument de stérilisation précité,
- la figure 13 représente une vue en coupe transversale d'une canule conforme à une variante de réalisation de la présente invention,
- la figure 14 représente une vue en coupe axiale longitudinale d'un instrument conforme à une variante de l'invention,
- la figure 15 représente une vue en coupe transversale de cet instrument selon le plan de coupe référencé XV-XV sur la figure 14, et
- la figure 16 représente une vue axiale en bout du même instrument selon la vue référencée XVI sur la figure 14.

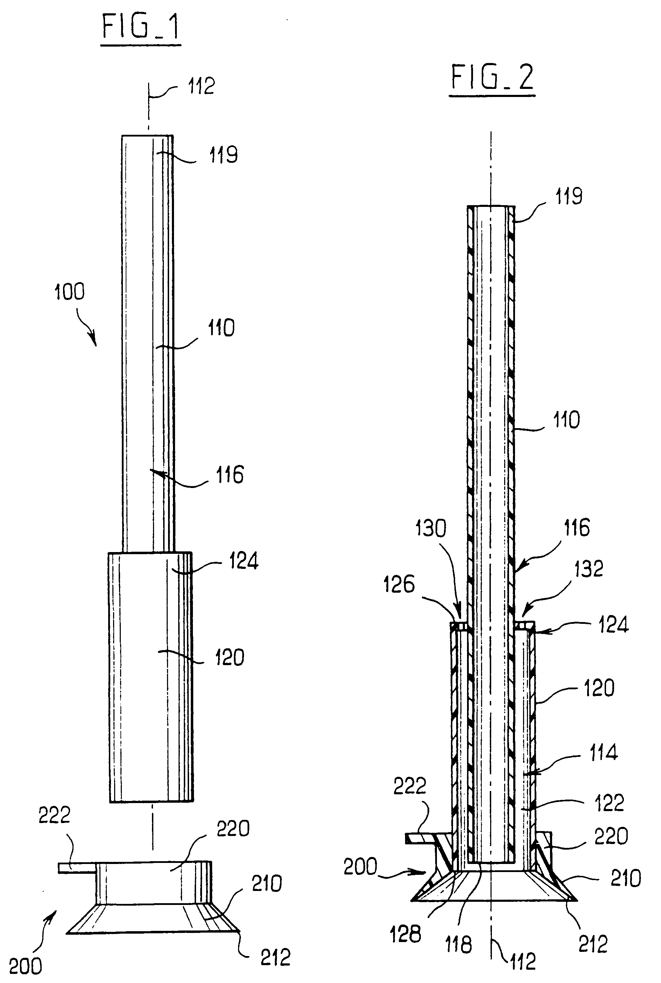

L'instrument chirurgical conforme à la présente invention, représenté sur les figures 1 à 3 annexées, comprend essentiellement une canule 100 équipée d'une ventouse 200 et deux aiguilles 300 et 400.

La canule 100 comprend un tube central cylindrique creux 110. Le tube 110 est centré sur un axe 112.

Le tube 110 est entouré sur sa partie distale 114 d'un fourreau externe 120. Il est ainsi défini entre le tube central 110 et le fourreau externe 120, une chambre annulaire 122.

La longueur du fourreau externe 120 est avantageusement de l'ordre de la moitié de celle du tube central 110. L'extrémité proximale 124 du fourreau 120 est ainsi située environ à la moitié de la longueur du tube 110.

Cette extrémité proximale 124 du fourreau 120 est raccordée de façon étanche sur la surface externe 116 du tube central 110, par une cloison d'obturation 126 qui s'étend transversalement à l'axe 112.

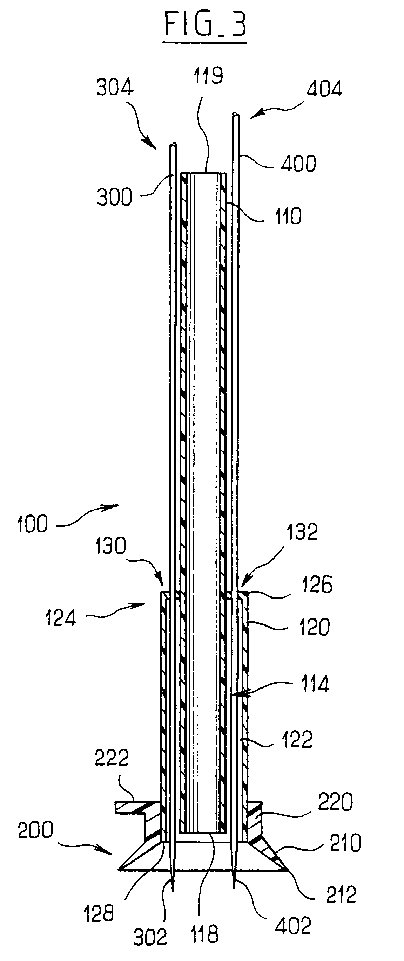

Cependant cette paroi est traversée par deux orifices 130 et 132 dont les axes sont parallèles à l'axe 112 et qui sont diamétralement opposés par rapport à l'axe 112. Le diamètre de ces orifices 130 et 132 est adapté pour permettre le déplacement à translation, parallèlement à l'axe 112, des aiguilles 300 et 400 engagées respectivement dans ces orifices comme on le voit sur la figure 3.

Les orifices 130 et 132 débouchent dans l'espace annulaire 122. Ainsi les aiguilles 300 et 400 ont leur extrémité distale située dans cet espace annulaire 122.

De préférence comme on le voit notamment sur la figure 2, l'extrémité distale 118 du tube central 110 est située en retrait de l'extrémité distale 128 du fourreau 120.

La ventouse 200 est placée sur l'extrémité distale 128 de la canule, plus précisément du fourreau 120.

La ventouse 200 peut être fixée à demeure sur l'extrémité distale 128 du fourreau 120.

Cependant selon le mode de réalisation particulier et non limitatif représenté sur les figures annexées, la ventouse 200 est indépendante de la canule 100 et adaptée pour être assemblée sur l'extrémité de celle-ci, à l'intérieur du corps humain sur le site d'utilisation. Pour celà la ventouse 200 et la canule 100 sont engagées successivement dans le corps humain, de préférence à travers un trocart.

Ainsi selon le mode de réalisation représenté sur les figures annexées la ventouse 200 comprend une jupe tronconique 210 qui se termine au niveau de son bord libre radialement externe par une lèvre annulaire souple 212. La jupe 210 se prolonge au niveau de son bord radialement interne par un manchon cylindrique 220 dont le diamètre interne est complémentaire du diamètre externe du fourreau 120. Le manchon 220 est coaxial à la jupe 210.

Pour faciliter la manipulation de la ventouse 200 à l'intérieur du corps en vue de son assemblage sur l'extrémité distale de la canule, le manchon 220 est avantageusement muni sur sa surface externe et au niveau de son bord libre, d'une languette 222 en saillie radialement par rapport à l'axe de la ventouse 200.

Les aiguilles 300 et 400 sont des aiguilles de ponction, rectilignes et creuses classiques.

Comme indiqué précédemment elles sont engagées libres de translation respectivement dans les orifices 130 et 132.

Les aiguilles 300 et 400 ainsi que la canule 200 doivent avoir une longueur suffisante pour rester accessibles à l'extérieur du corps tout en étant en contact avec le kyste à traiter dans le corps humain.

A titre d'exemple non limitatif, la longueur du tube central 110 peut être de l'ordre de 16 cm, la longueur du fourreau 120 peut être de l'ordre de 6 cm, le diamètre externe du tube central 110 peut être de l'ordre de 12 mm, tandis que le diamètre externe du fourreau 120 est de l'ordre de 18 mm.

Le diamètre externe de la lèvre 212 de la ventouse 200 peut être typiquement de l'ordre de 5 à 8 cm. Plus précisément il est prévu de préférence plusieurs tailles de ventouse 200 afin de permettre au chirurgien de choisir la ventouse la mieux adaptée à l'intervention en fonction du kyste à traiter et du patient. A titre d'exemple non limitatif on peut ainsi prévoir trois tailles de ventouse 200, soit une ventouse de petite taille d'un diamètre de l'ordre de 3 à 5 cm, une ventouse de taille moyenne d'un diamètre de l'ordre de 5 à 7 cm et une ventouse de grande taille d'un diamètre de l'ordre de 7 à 9 cm.

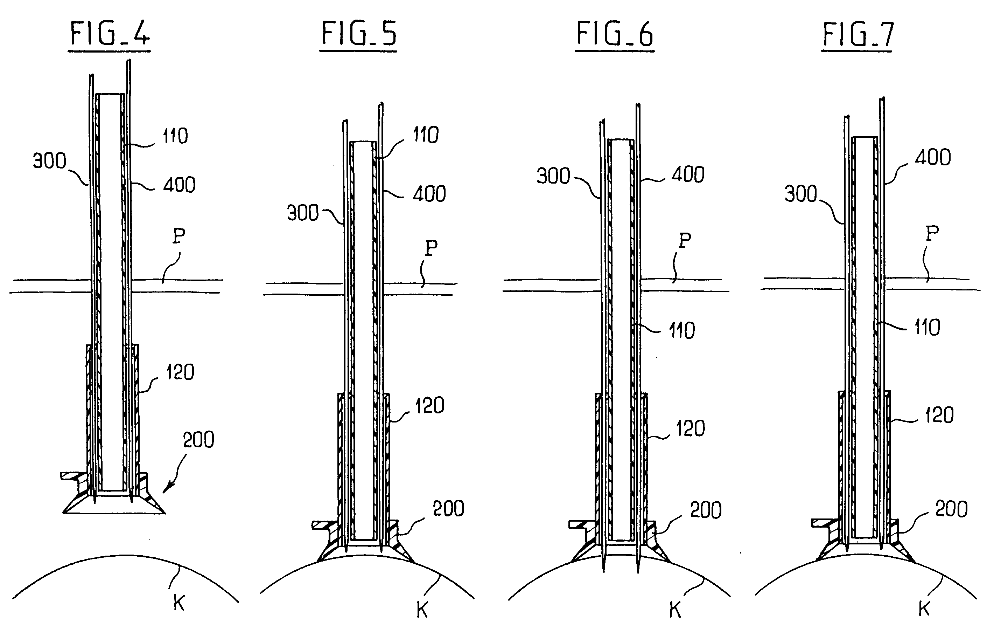

On va maintenant décrire le procédé d'utilisation de l'instrument précité en regard des figures 4 à 7. Sur ces figures le kyste à traiter est référencé K, tandis que la peau du patient est référencée P.

Sur les figures 4 à 7 on a omis, pour simplifier l'illustration, le trocart d'introduction de la canule 100. Un tel trocart est en effet classique en lui même. Par ailleurs ce trocart n'est utile que dans la phase d'introduction de la canule, il ne joue pas de rôle particulier par la suite et peut même être retiré lorsque la canule 100 a été positionnée sur le site d'utilisation.

Comme représenté sur la figure 4, après avoir introduit la ventouse 200 et la canule 100 dans le corps humain, la ventouse 200 est assemblée sur l'extrémité distale de la canule 100.

Puis comme représenté sur la figure 5, la ventouse 200 est portée au contact du kyste K au niveau de la zone du dome saillant ou partie affleurante choisie pour la ponction.

L'extrémité proximale 119 du tube 110 est ensuite reliée à une source en dépression. Ainsi l'ensemble du volume interne de la canule 100 et de la ventouse 200 est placé en dépression ou pression négative par rapport à l'environnement. De ce fait la ventouse 200 adhère étroitement au kyste K pour confiner la zone de ponction. D'autre part grâce à la dépression créée dans la ventouse 200 on évite tout risque de fuite de liquide hydatique.

Au cours des étapes antérieures, les aiguilles 300 et 400 ont leur extrémité distale 302 et 402 en retrait dans la ventouse 200 ou dans la chambre annulaire 122 de la canule 100.

En revanche comme représenté sur la figure 6, les aiguilles 300 et 400 sont ensuite déplacées vers le kyste K de sorte que leur extrémité distale 302 et 402 pénètre dans le kyste K.

Les aiguilles 300 et 400 peuvent alors être utilisées pour vider le kyste K de son liquide hydatique et remplir celui-ci d'un liquide de stérilisation.

Au cours de cette étape, de préférence, on peut utiliser une aiguille 300 pour retirer le liquide infecté hors du kyste K, par aspiration, et l'autre aiguille 400 pour remplir simultanément le kyste K en liquide de stérilisation. Plus précisément on veille de préférence à réinjecter dans le kyste K une quantité de liquide de stérilisation égale à la quantité de liquide retiré, de sorte que le volume du kyste K reste sensiblement constant pour garantir notamment une bonne adhérence de la ventouse 200.

Une fois le kyste K correctement stérilisé, comme représenté sur la figure 7, les extrémités distales 302 et 402 des aiguilles 300 et 400 sont rétractées dans la ventouse 200 ou dans la chambre annulaire 122.

Bien entendu au cours des étapes précédentes la dépression est maintenue dans la canule grâce aux moyens reliés à l'extrémité proximale du tube 100.

On peut alors procéder à la stérilisation du volume interne de la ventouse 200 et de la canule 100 pour traiter le liquide qui aurait pu s'écouler dans ce volume sur la périphérie des aiguilles 300 et 400.

Pour celà la dépression doit être maintenue dans la ventouse 200 grâce au tube 110 ou à l'une au moins des aiguilles 300 ou 400, tandis que les autres moyens, à savoir l'une au moins des aiguilles 300 ou 400 ou le tube 110 peuvent être utilisés pour opérer la stérilisation de la ventouse 200 et de la canule 100 par aspiration et remplissage à l'aide d'un liquide de stérilisation. Il est important de veiller à ce que la ventouse reste toujours en pression négative par rapport à son environnement.

Cette étape peut également être mise à profit pour parfaire la stérilisation de la membrane du kyste K aprés avoir pratiqué une ouverture dans la membrane au niveau de la zone de celle-ci circonscrite par la ventouse, à l'aide d'un outil de coupe engagé par le tube central 110.

En rétractant les extrémités distales des aiguilles 300 et 400 en deçà des extrémités distales du tube 110 et du fourreau 120, soit dans la chambre annulaire 120, on évite tout risque d'obturation des aiguilles 300 et 400 par la membrane du kyste K. En effet cette membrane prend appui contre les extrémités distales du tube central 110 et du fourreau 120 et ne peut atteindre les extrémités des aiguilles.

La membrane du kyste K peut ensuite être éliminée par tous moyens, de préférence par aspiration à travers le tube central 110.

Les moyens permettant de relier les extrémités proximales 119 de la canule 110 et 304 ou 404 des aiguilles, à des moyens à dépression, d'aspiration ou de remplissage en matériau de stérilisation, ne sont pas représentés sur les figures annexées pour simplifier l'illustration.

En pratique les moyens à dépression peuvent être formés de simples flacons sous vide reliès, par une tubulure, à l'extrémité proximale de la canule 110 ou des aiguilles.

Les moyens d'aspiration et/ou de remplissage peuvent être formés de seringues raccordées aux mêmes extrémités.

Bien entendu la présente invention n'est pas limitée au mode de réalisation particulier qui vient d'être décrit, mais s'étend à toutes variantes conformes à la revendication 1 annexée.

Ainsi la canule 100 peut être formée par moulage d'une pièce unique ou encore par assemblage de différentes pièces, par exemple par assemblage d'un fourreau 120 sur un tube central 110.

Le cas échéant on peut également prévoir des moyens d'étanchéité au niveau des orifices 130 et 132 autour des aiguilles 300 et 400.

La ventouse 200 peut être munie de soufflets annulaires pour améliorer sa souplesse.

Dans le cadre d'une intervention dans une cavité du corps humain dilatée par insufflation d'air, comme celà est désormais classique pour les interventions sous endoscopie, notamment les interventions dans l'abdomen, il est de préférence prévu des moyens d'obturation amovibles sur les extrémités proximales de la canule 100 et des aiguilles 300 et 400, pour éviter une fuite d'air lors de l'introduction de l'instrument dans le corps humain. Pour celà on peut par exemple prévoir de relier les extrémités proximales de la canule et des aiguilles aux moyens à dépression, aux moyens d'aspiration ou aux moyens d'injection de liquide de stérilisation, par l'intermédiaire de vannes controlées manuellement.

Selon une caractéristique avantageuse de la présente invention, le matériau composant la canule 100 est optiquement transparent pour permettre de contrôler aisément la nature des fluides et de la membrane transitant par celle-ci.

Le cas échéant on peut prévoir des moyens formant butée élastique pour indexer les aiguilles 300 et 400 par rapport à la canule 100 respectivement dans leur deux positions rétractée et en saillie.

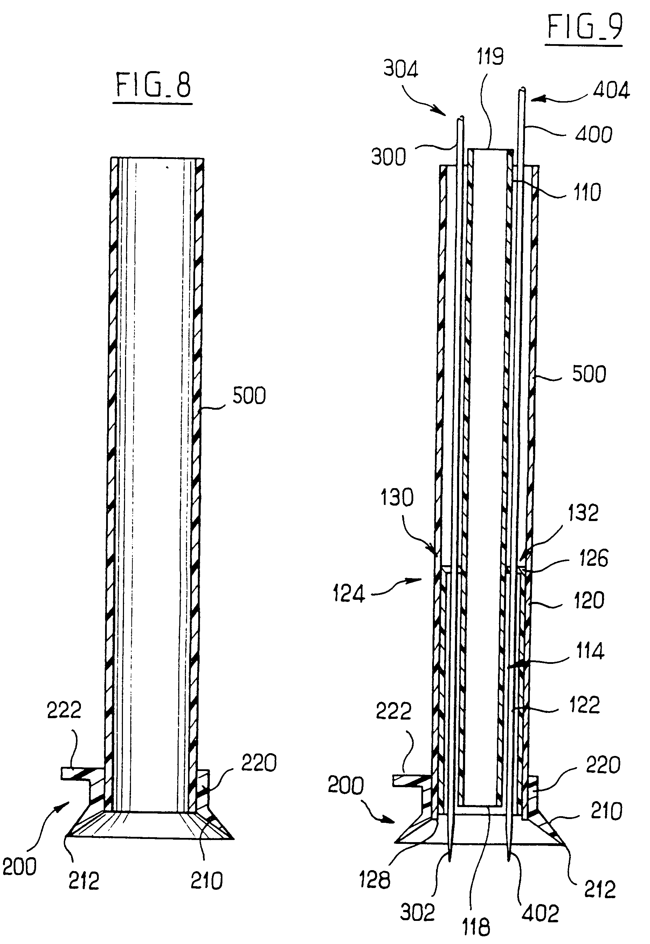

Selon une autre variante de réalisation représentée sur les figures 8 et 9 annexées, la canule 100 précédemment décrite peut être placée dans un corps tubulaire externe de canule 500. Il s'agit de préférence d'un tube cylindrique dont le diamètre interne est adapté étroitement au diamètre externe de la canule 100 tout en autorisant un déplacement à translation entre ceux-ci.

Comme on le voit sur les figures 8 et 9, dans ce cas la ventouse 200 n'est plus fixée sur l'extrémité distale du fourreau 120, mais sur l'extrémité distale du corps externe 500 de la canule.

Plus précisément il peut s'agir là encore d'une ventouse 200 fixée à demeure sur l'extrémité distale du corps externe 500 ou d'une ventouse 200 indépendante du corps 500 et fixée sur celui-ci au moment de l'intervention, sur le site d'utilisation.

Le corps externe tubulaire 500 de la canule doit être introduit dans le corps humain avec la canule 100. Les opérations de stérilisation restent conformes aux modalités précédemment décrites. Cependant une fois la stérilisation de la membrane achevée, la canule centrale 100 peut être retirée, seul le corps externe tubulaire 500 de la canule restant alors en place, afin de disposer d'une section plus importante pour aspirer la membrane du kyste dans de bonnes conditions.

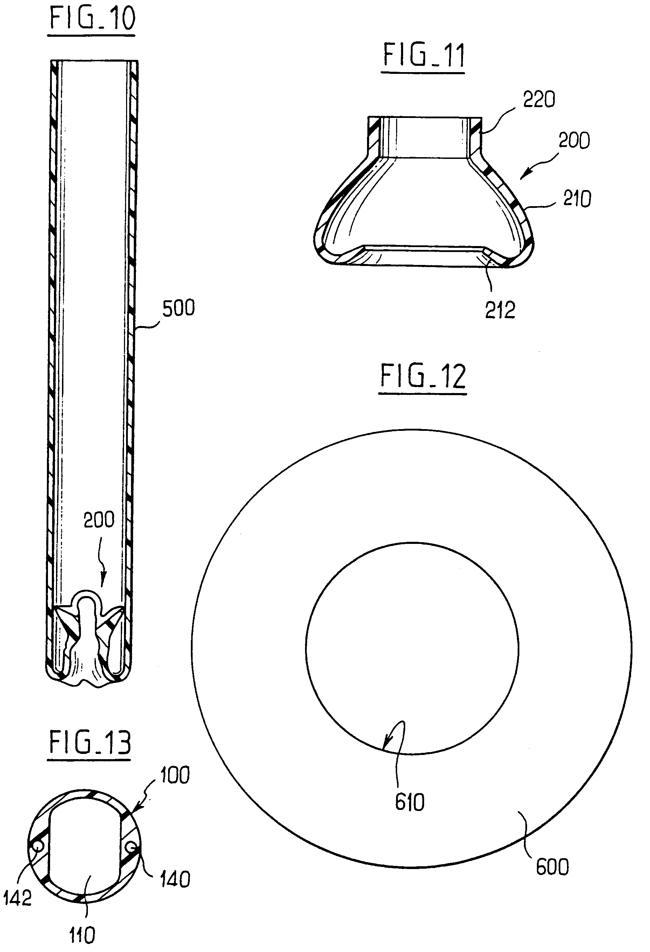

On a représenté sur la figure 10 une variante de réalisation d'un tel corps externe tubulaire de canule 500 équipé à demeure d'une ventouse 200 adaptée pour être repliée avant utilisation à l'intérieur de l'extrémité distale du corps 500. Aprés introduction du corps de canule 500 dans le corps humain, il suffit d'engager la canule centrale 100 dans le corps tubulaire 500 pour déployer la ventouse 200.

On peut de même prévoir une telle ventouse 200 repliée avant utilisation à l'intérieur de l'extrémité distale du tube central 110, dans le cadre du mode de réalisation des figures 1 à 3. Pour déployer la ventouse 200 il suffit alors d'engager un mandrin ou tout moyen équivalent dans le tube central 110.

On a représenté sur la figure 11 une variante de réalisation de ventouse 200 dans laquelle la lèvre souple 212 est repliée radialement vers l'intérieur de la ventouse. Cette variante permet d'une part d'augmenter la surface de la ventouse en contact avec la membrane du kyste K. D'autre part elle permet de définir ainsi un rebord anti-fuite.

Enfin on représenté sur la figure 12 une plaque 600 de matériau absorbant susceptible d'être utilisée en combinaison avec l'instrument de stérilisation précité.

Cette plaque 600 est adaptée pour être introduite en position repliée, par l'intermédiaire du trocart, dans le corps humain et pour se déployer ensuite dans le corps humain afin d'être placée au contact du kyste K.

La plaque 600 possède une ouverture centrale 610 de dimensions complémentaires de la ventouse 200. La plaque 600 est destinée à entourer la ventouse 200 lors de l'intervention. Elle est imbibée d'un liquide scolicide pour améliorer encore la sécurité de l'intervention.

Selon le mode de réalisation représenté sur la figure 12 annexée, le contour de la plaque 600 est circulaire. Toutefois l'invention n'est pas limitée à cette géométrie. Le contour de la plaque 600 peut même être adapté aisément par le chirurgien au moment de l'intervention à la géométrie du dome saillant du kyste K.

Selon une autre variante de réalisation la canule 100 peut possèder un diamètre externe constant sur toute sa longueur. Le décrochement que l'on voit sur les figures 1 à 3 au niveau de l'extrémité proximale du fourreau 120 est alors supprimé. On peut ainsi prévoir comme représenté sur la figure 13, une canule 100 comprenant deux conduits longitudinaux parallèles 140, 142 recevant les aiguilles 300 et 400 à translation et débouchant dans une chambre annulaire distale ou des moyens équivalents comparables à la chambre annulaire 122 précédemment décrite. Dans ce cas la section du conduit central libre 110 utilisé pour aspirer la membrane du kyste K peut être non circulaire afin d'exploiter au mieux le volume de la canule disponible.

Selon une autre variante, on peut prévoir de relier les moyens à dépression, à l'origine, à la chambre annulaire 122 et non point au tube central 110.

Comme indiqué précédemment l'instrument conforme à la présente invention s'avère particulièrement avantageux pour une intervention sous endoscopie. Toutefois l'instrument conforme à la présente invention peut également être utilisé à ciel ouvert.

Par ailleurs la présente invention s'applique en particulier aux interventions dans le thorax et dans l'abdomen. Toutefois l'instrument conforme à la présente invention peut être utilisé pour des interventions dans d'autres parties du corps humain.

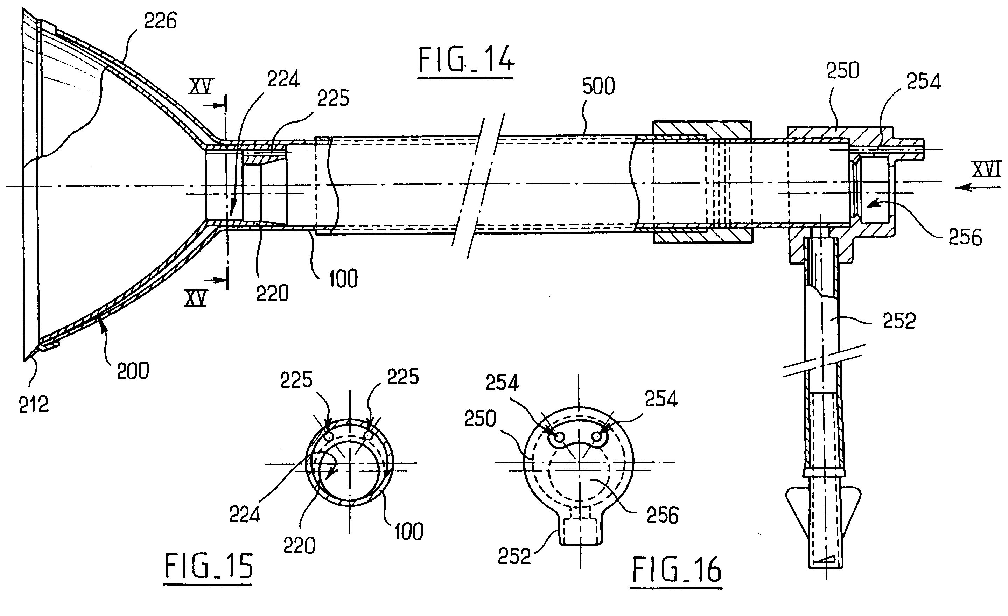

On retrouve sur les figures 14 à 16 un instrument comprenant une canule centrale 100 associée à une ventouse 200, et un corps tubulaire externe 500.

La canule 100 est formée de préférence d'un tube rectiligne cylindrique en matériau optiquement transparent apte à coulisser longitudinalement à l'intérieur du corps externe 500.

La ventouse 200 est prévue à l'extrémité distale de la canule 100. La ventouse 200 est formée de préférence d'une cloche en matériau silicone. Elle a la forme générale d'un tronc de cône ou d'une demi-sphère. La ventouse 200 est munie de préférence d'une lèvre annulaire 212 à sa base la plus large opposée à la canule 100. A son sommet, la ventouse 200 est munie d'un embout 220 engagé dans l'extrémité distale de la canule 100. Cet embout 200 possède un canal longitudinal traversant 224. Il possède également deux passages longitudinaux parallèles 225 conçus pour recevoir à translation et guider les aiguilles de ponction 300 et 400. Ces dernières ne sont pas représentées sur les figures 14 à 16 pour simplifier l'illustration. Selon une caractéristique importante de la variante des figures 14 à 16, la ventouse 200 en silicone est renforcée par des baleines ou équivalents 226. Ces baleines 226 sont formées de tout matériau approprié, de préférence en matière plastique. Elle sont conçues pour faciliter le déploiement de la ventouse 200. Les baleines 226 peuvent être venues de matière à partir de l'extrémité distale de la canule 100 comme représenté sur la figure 14.

En variante cependant les baleines 226 peuvent être formées de pièces séparées, rapportées sur la ventouse 200, par exemple collées ou surmoulées.

A son extrémité proximale, la canule 100 possède un chapeau 250. Le chapeau 250 possède un ajutage transversal 252 conçu pour être relié à des moyens à dépression, pour soumettre le volume interne de la canule 100 à une dépression contrôlée. Le chapeau 250 possède de plus deux canaux 254 respectivement alignés sur les canaux 225 précités pour recevoir et guider les aiguilles 300, 400. Enfin, le chapeau 250 comprend des moyens d'obturation amovibles 256, constitués par exemple d'une paroi transversale fendue en croix. De tels moyens d'obturation 256 permettent l'introduction de tout ustensile adéquat à l'intérieur de la canule 100, notamment tout ustensile d'aspiration pour l'élimination de la poche du kyste après ponction.

L'embout 220 peut être fixé sur l'extrémité distale de la canule 100 par tout moyen approprié, par exemple par collage.

Le corps extérieur 500 est formé de préférence d'un tube transparent.

Pour utiliser l'instrument illustré sur les figures 14 à 16, on procède essentiellement comme suit. La ventouse 200 est rétractée à l'intérieur du corps externe 500. Celui-ci est approché du lieu d'utilisation. La ventouse 200 est extraite du corps extérieur 500 par coulissement de la canule 100 à l'intérieur de ce dernier. Le déploiement en cloche de la ventouse 200 est facilité par la présence des baleines 226.

Pour le reste, le collage de la ventouse 200 sur la poche du kyste, par mise en dépression de la canule 100 puis la ponction et l'élimination du kyste à l'aide des aiguilles 300, 400 sont opérés comme indiqué précédemment.

## Revendications

1. Instrument chirurgical pour le traitement d'une collection liquidienne, en particulier pour la stérilisation et l'aspiration d'un kyste hydatique, comprenant :
- une canule (100) pourvue d'une ventouse (200) à son extremité distale (118, 218) et conçue pour être raccordée à une source à dépression à son extrémité proximale (119),
- et au moins une aiguille creuse de ponction (300, 400), dont l'extrémité distale (302, 402) est entourée par la canule (100), apte à être déplacée à translation par rapport à la canule (100) entre une première position dans laquelle la pointe (302, 402) de l'aiguille (300, 400) est en retrait dans la ventouse (200) ou la canule (100) et une seconde position, de ponction, dans laquelle la pointe (302, 402) de l'aiguille fait saillie au delà de la ventouse (200), caractérisé par le fait qu'il comprend deux aiguilles creuses (300, 400) et au moins un canal opérateur (110) séparé des aiguilles et adapté pour recevoir au moins un outil d'intervention dans la canule.

2. Instrument selon la revendicaiton 1, caractérisé par le fait que la ventouse (200) comprend une lèvre souple annulaire (212).

3. Instrument selon l'une des revendications 1 ou 2, caractérisé par le fait que la canule (100) comprend un tube central (110) entouré au niveau de sa partie distale par un fourreau (120).

4. Instrument selon la revendication 3, caractérisé par le fait que l'extrémité proximale du fourreau (120) est reliée de façon étanche à la surface extérieure du tube central (110).

5. Instrument selon l'une des revendications 3 ou 4, caractérisé par le fait que l'extrémité proximale (124) du fourreau (120) est munie de deux orifices (130, 132) recevant respectivement une aiguille (300, 400) libre de translation.

6. Instrument selon l'une des revendications 3 à 5, caractérisé par le fait que les aiguilles (300, 400) sont placées dans la chambre annulaire (122) formée entre le tube central (110) et le fourreau (120).

7. Instrument selon l'une des revendications 1 à 6, caractérisé par le fait que l'extrémité distale (118) du tube central (110) est placée en retrait de l'extrémité distale (128) du fourreau (120).

8. Instrument selon l'une des revendications 1 à 7, caractérisé par le fait que la ventouse (200) est solidaire à demeure de l'extrémité distale de la canule (100).

9. Instrument selon l'une des revendications 1 à 7, caractérisé par le fait que la ventouse (200) est indépendante de la canule (100) et adaptée pour être fixée sur l'extrémité distale de celle-ci sur le site d'utilisation, dans le corps humain.

10. Instrument selon la revendication 9, caractérisé par le fait que la ventouse (200) comprend une jupe tronconique (210) pourvue d'une lèvre souple (212), laquelle jupe est prolongée par un manchon (220) muni d'une languette radiale (222) de manipulation.

11. Instrument selon l'une des revendications 1 à 10 caractérisé par le fait que la ventouse (200) comprend des soufflets annulaires.

12. Instrument selon l'une des revendications 1 à 11, caractérisé par le fait qu'il comprend des moyens d'obturation amovibles pour les extrémités proximales de la canule (100) et de chaque aiguille (300, 400).

13. Instrument selon l'une des revendications 1 à 12, caractérisé par le fait le matériau composant la canule (100) est optiquement transparent.

14. Instrument selon l'une des revendications 1 à 13, caractérisé par le fait qu'il comprend des moyens d'indexation de chaque aiguille (300, 400) par rapport à la canule (100), respectivement dans ses deux positions rétractée et en saillie.

15. Instrument selon l'une des revendications 1 à 14, caractérisé par le fait qu'il comprend un corps tubulaire externe de canule (500) qui reçoit libre de translation un élément de canule (100) définissant un conduit central (110) et adapté pour recevoir au moins une aiguille creuse de ponction (300, 400).

16. Instrument selon la revendication 15, caractérisé par le fait que le corps tubulaire externe (500) est séparable de l'élément de canule (100).

17. Instrument selon l'une des revendications 15 ou 16, caractérisé par le fait que la ventouse (200) est fixée à demeure sur l'extrémité distale du corps tubulaire externe (500).

18. Instrument selon l'une des revendications 15 ou 16, caractérisé par le fait que la ventouse (200) est adaptée pour être fixée de façon amovible sur l'extrémité distale du corps tubulaire externe (500).

19. Instrument selon l'une des revendications 1 à 18 caractérisé par le fait que la ventouse (200) est repliée avant utilisation à l'intérieur de l'extrémité distale de la canule (100, 500) et adaptée pour être déployée à l'extérieur de celle-ci après introduction dans le corps humain.

20. Instrument selon l'une des revendications 1 à 19 caractérisé par le fait que la ventouse (200) comprend une lèvre souple (212) repliée radialement vers l'inttérieur de la ventouse (200).

21. Instrument selon l'une des revendications 1 à 20 caractérisé par le fait que la canule (100) possède une section sensiblement constante sur toute sa longueur.

22. Instrument selon l'une des revendications 1 à 21 caractérisé par le fait que la longueur de la canule (100) est de l'ordre de 16 cm.

23. Instrument selon l'une des revendications 1 à 22 caractérisé par le fait que le diamètre de la ventouse (200) est de l'ordre de 3 à 9 cm.

24. Instrument selon l'une des revendications 1 à 23 caractérisé par le fait qu'il est prévu des ventouses (200) de différentes tailles pour permettre au chirurgien d'adapter la ventouse à l'intervention, en fonction de la collection à traiter et du patient.

25. Instrument selon l'une des revendications 1 à 24 caractérisé par le fait qu'il comprend en outre une plaque (600) en matériau absorbant comportant une ouverture (610) adaptée pour être engagée sur la ventouse (200).

26. Instrument selon l'une des revendications 1 à 25, caractérisé par le fait que la ventouse (200) est associée à des baleines (226) de rigidification facilitant son déploiement.

27. Instrument selon la revendication 26, caractérisé par le fait que les baleines (226) sont venues de matière avec la canule (100).

28. Instrument selon l'une des revendications 26 ou 27, caractérisé par le fait que la canule (100) est montée à coulissement dans un corps extérieur (500) et la ventouse (200) solidaire de la canule (100) est adaptée pour être rétractée à l'intérieur du corps extérieur (500).

## Claims

1. A surgical instrument for treating a watery gathering, in particular for sterilization and aspiration of a hydatid cyst, the instrument comprising:
a cannula (100) provided with a suction cup (200) at its distal end (118, 218) and designed to be connected to a suction source at its proximal end (119); and
at least one hollow puncture needle (300, 400) whose distal end (302, 402) is surrounded by the cannula (100), which needle is suitable for being displaced in translation relative to the cannula (100) between a first position in which the tip (302, 402) of the needle (300, 400) is set back inside the suction cup (200) or the cannula (100), and a puncturing, second position in which the tip (302, 402) of the needle projects beyond the suction cup (200), the instrument being characterized by the fact that it includes two hollow needles (300, 400) and at least one operating channel (110) in the cannula separated from the needles and adapted to receive at least one intervention tool.

2. An instrument according to claim 1, characterized by the fact that the suction cup (200) includes a flexible annular lip (212).

3. An instrument according to claim 1 or 2, characterized by the fact that the cannula (100) has a central tube (110) surrounded at its distal end by a sheath (120).

4. An instrument according to claim 3, characterized by the fact that the proximal end of the sheath (120) is connected in sealed manner to the outside surface of the central tube (110).

5. An instrument according to claim 3 or 4, characterized by the fact that the proximal end (124) of the sheath (120) is provided with two orifices (130, 132) each receiving a respective needle (300, 400) that is free to move in translation.

6. An instrument according to any one of claims 3 to 5, characterized by the fact that the needles (300, 400) are placed in the annular chamber (122) formed between the central tube (110) and the sheath (120).

7. An instrument according to any one of claims 1 to 6, characterized by the fact that the distal end (118) of the central tube (110) is set back from the distal end (128) of the sheath (120).

8. An instrument according to any one of claims 1 to 7, characterized by the fact that the suction cup (200) is permanently secured to the distal end of the cannula (100).

9. An instrument according to any one of claims 1 to 7, characterized by the fact that the suction cup (200) is independent from the cannula (100) and is adapted to be fixed to the distal end thereof at the site of use, inside the human body.

10. An instrument according to claim 9, characterized by the that the suction cup (200) includes a frustoconical skirt (210) provided with a flexible lip (212), which skirt is extended by a sleeve (220) provided with a radial tongue (222) for handling purposes.

11. An instrument according to any one of claims 1 to 10, characterized by the fact that the suction cup (200) includes annular bellows.

12. An instrument according to any one of claims 1 to 11, characterized by the fact that it includes removable closure means for the proximal ends of the cannula (100) and of each needle (300, 400).

13. An instrument according to any one of claims 1 to 12, characterized by the fact that the cannula (100) is made from optically transparent material.

14. An instrument according to any one of claims 1 to 13, characterized by the fact that it includes means for indexing each needle (300, 400) relative to the cannula (100), each in respective retracted and extended positions.

15. An instrument according to any one of claims 1 to 14, characterized by the fact that it comprises a cannula outer tubular body (500) that receives a cannula element (100) free to move in translation which defines a central duct (110) and adapted to receive at least one hollow puncture needle (300, 400).

16. An instrument according to claim 15, characterized by the fact that the outer tubular body (500) is separable from the cannula element (100).

17. An instrument according to claim 15 or 16, characterized by the fact that the suction cup (200) is permanently secured to the distal end of the outer tubular body (500).

18. An instrument according to claim 15 or 16, characterized by the fact that the suction cup (200) is adapted to be removably fixed to the distal end of the outer tubular body (500).

19. An instrument according to any one of claims 1 to 18, characterized by the fact that the suction cup (200) is folded up prior to use within the distal end of the cannula (100, 500) and is adapted to be deployed therefrom after insertion into the human body.

20. An instrument according to any one of claims 1 to 19, characterized by the fact that the suction cup (200) has a flexible lip (212) that is radially folded towards the inside of the suction cup (200).

21. An instrument according to any one of claims 1 to 20, characterized by the fact that the section of the cannula (100) is substantially constant along its entire length.

22. An instrument according to any one of claims 1 to 21, characterized by the fact that the length of the cannula (100) is about 16 cm.

23. An instrument according to any one of claims 1 to 22, characterized by the fact that the diameter of the suction cup (200) is about 3 cm to 9 cm.

24. An instrument according to any one of claims 1 to 23, characterized by the fact that suction cups (200) of different sizes are provided to enable the surgeon to adapt the suction cup to the intervention, as a function of the gathering to be treated and as a function of the patient.

25. An instrument according to any one of claims 1 to 24, characterized by the fact that it further includes a piece (600) of absorbent material with an opening (610) adapted to be engaged on the suction cup (200).

26. An instrument according to any one of claims 1 to 25, characterized by the fact that the suction cup (200) is associated with stiffening ribs (226) that facilitate deployment thereof.

27. An instrument according to claim 26, characterized by the fact that the ribs (226) are integrally molded with the cannula (100).

28. An instrument according to claim 26 or 27, characterized by the fact that the cannula (100) is slidably mounted in an outer body (500), and the suction cup (200) secured to the cannula (100) is adapted to be retracted into the outer body (500).

## Patentansprüche

1. Chirurgisches Instrument zur Behandlung einer Flüssigkeitsansammlung, insbesondere zur Sterilisation und Aspiration einer Wasser enthaltenden Zyste, mit:
- einer Kanüle (100), die an ihrem distalen Ende (118, 218) mit einem Saugnapf (200) versehen und an ihrem proximalen Ende (119) zum Anschluß an eine Unterdruckquelle vorgesehen ist,
- und mindestens einer Punktions-Hohlnadel (300, 400), deren distales Ende (302, 402) von der Kanüle (100) umgeben ist und die zu einer Translationsverschiebung in bezug auf die Kanüle (100) zwischen einer ersten Position, in welcher die Spitze (302, 402) der Nadel (300, 400) in den Saugnapf (200) oder in die Kanüle (100) zurückgezogen ist und einer zweiten Punktionsposition, in welcher die Spitze (302, 402) der Nadel (300, 400) aus dem Saugnapf (200) herautritt, geeignet ist, dadurch gekennzeichnet, daß sie zwei Hohlnadeln (330, 400) sowie mindestens einen von den Nadeln getrennten Operationskanal (110) aufweist, der zur Aufnahme mindestens eines Operationswerkzeuges in der Kanüle geeignet ist.

2. Instrument nach Anspruch 1 , dadurch gekennzeichnet, daß der Saugnapf (200) eine umlaufende flexible Lippe (212) aufweist.

3. Instrument nach einem der Ansprüche 1 oder 2, dadurch gekennzeichnet, daß die Kanüle (100) ein Zentralrohr (110) aufweist, welches an seinem distalen Teil von einer Buchse (120) umgeben ist.

4. Instrument nach Anspruch 3, dadurch gekennzeichnet, daß das proximale Ende der Buchse (120) dicht mit der Außenfläche des Zentralrohres (110) verbunden ist.

5. Instrument nach einem der Ansprüche 3 oder 4, dadurch gekennzeichnet, daß das proximale Ende (124) der Buchse (120) mit zwei Öffnungen (130, 132) versehen ist, welche jeweils eine Nadel (300, 400) aufnehmen und eine Translationsbewegung derselben zulassen.

6. Instrument nach einem der Ansprüche 3 bis 5, dadurch gekennzeichnet, daß die Nadeln (300, 400) in der ringförmigen Kammer (122) angeordnet sind, welche zwischen dem Zentralrohr (110) und der Buchse (120) ausgebildet ist.

7. Instrument nach einem der Ansprüche 1 bis 6, dadurch gekennzeichnet, daß das distale Ende (118) des Zentralrohres (110) gegenüber dem distalen Ende (128) der Buchse (120) zurückgesetzt angeordnet ist.

8. Instrument nach einem der Ansprüche 1 bis 7, dadurch gekennzeichnet, daß der Saugnapf (200) dauerhaft am distalen Ende der Kanüle (100) befestigt ist.

9. Instrument nach einem der Ansprüche 1 bis 7, dadurch gekennzeichnet, daß der Saugnapf (200) von der Kanüle (100) unabhängig sowie geeignet ist, am Einsatzort im menschlichen Körper an deren Ende befestigt zu werden.

10. Instrument nach Anspruch 9, dadurch gekennzeichnet, daß der Saugnapf (200) einen mit einer flexiblen Lippe (212) versehenen kegelstumpfförmigen Mantel (210) aufweist, der sich in einer Hülse (220) fortsetzt, welche mit einer radialen Handhabungszunge (222) versehen ist.

11. Instrument nach einem der Ansprüche 1 bis 10, dadurch gekennzeichnet, daß der Saugnapf (200) ringförmige Faltenbälge aufweist.

12. Instrument nach einem der Ansprüche 1 bis 11, dadurch gekennzeichnet, daß es abnehmbare Verschlußeinrichtungen für die proximalen Enden der Kanüle (100) und jede der Nadeln (300, 400) aufweist.

13. Instrument nach einem der Ansprüche 1 bis 12, dadurch gekennzeichnet, daß das die Kanüle (100) bildende Material optisch durchsichtig ist.

14. Instrument nach einem der Ansprüche 1 bis 13, dadurch gekennzeichnet, daß es Rasteinrichtungen für jede der Nadeln (300, 400) in bezug auf die Kanüle (100) beziehungsweise für die zurückgezogene und die vorgeschobene Position aufweist.

15. Instrument nach einem der Ansprüche 1 bis 14, dadurch gekennzeichnet, daß es ein Kanülen-Außenrohr (500) aufweist, welches ein Kanülenelement (100), das einen Zentralkanal (110) begrenzt, aufnimmt sowie dessen freie Translation zuläßt und geeignet ist, mindestens eine Punktions-Hohlnadel (300, 400) aufzunehmen.

16. Instrument nach Anspruch 15, dadurch gekennzeichnet, daß das Außenrohr (500) vom Kanülenelement (100) abnehmbar ist.

17. Instrument nach einem der Ansprüche 15 oder 16, dadurch gekennzeichnet, daß der Saugnapf (200) dauerhaft am distalen Ende des Außenrohres (500) befestigt ist.

18. Instrument nach einem der Ansprüche 15 oder 16, dadurch gekennzeichnet, daß der Saugnapf (200) abnehmbar am distalen Ende des Außenrohres (500) befestigt ist.

19. Instrument nach einem der Ansprüche 1 bis 18, dadurch gekennzeichnet, daß sich der Saugnapf (200) vor dem Einsatz zusammengefaltet im Inneren des distalen Endes der Kanüle (100, 500) befindet und derart ausgebildet ist, daß er nach der Einführung in den menschlichen Körper außerhalb der Kanüle entfaltet wird.

20. Instrument nach einem der Ansprüche 1 bis 19, dadurch gekennzeichnet, daß der Saugnapf (200) eine radial zum Inneren des Saugnapfes (200) hin gefaltete weiche Lippe (212) aufweist.

21. Instrument nach einem der Ansprüche 1 bis 20, dadurch gekennzeichnet, daß die Kanüle (100) über ihre gesamte Länge einen genau konstanten Querschnitt besitzt.

22. Instrument nach einem der Ansprüche 1 bis 21, dadurch gekennzeichnet, daß die Länge der Kanüle (100) die Größenordnung von 16 cm hat.

23. Instrument nach einem der Ansprüche 1 bis 22, dadurch gekennzeichnet, daß der Durchmesser des Saugnapfes (200) die Größenordnung von 3 bis 9 cm hat.

24. Instrument nach einem der Ansprüche 1 bis 23, dadurch gekennzeichnet, daß Saugnäpfe (200) verschiedener Größen vorgesehen sind, um es dem Chirurgen zu ermöglichen, den Saugnapf in Abhängigkeit von der zu behandelnden Flüssigkeitsansammlung beim Patienten an die Operation anzupassen.

25. Instrument nach einem der Ansprüche 1 bis 24, dadurch gekennzeichnet, daß es eine Platte (600) aus saugfähigem Material mit einer Öffnung (610) aufweist, die zum Aufstecken auf den Saugnapf (200) geeignet ist.

26. Instrument nach einem der Ansprüche 1 bis 25, dadurch gekennzeichnet, daß der Saugnapf (200) zur Erleichterung seiner Entfaltung mit Verstärkungsstreben (226) versehen ist.

27. Instrument nach Anspruch 26, dadurch gekennzeichnet, daß die Verstärkungsstreben (226) stoffschlüssig mit der Kanüle (100) verbunden sind.

28. Instrument nach einem der Ansprüche 26 oder 27, dadurch gekennzeichnet, daß die Kanüle (100) verschiebbar in einem Außenrohr (500) montiert und der fest mit der Kanüle (100) verbundene Saugnapf (200) geeignet ist, in das Innere des Außenrohres (500) zurückgezogen zu werden.
